# EUROPÄISCHE PATENTSCHRIFT

(11) **EP 3 313 815 B1**
(45) Veröffentlichungstag und Bekanntmachung des Hinweises auf die Patenterteilung: **31.07.2019**
(21) Anmeldenummer: 16731940.9
(22) Anmeldetag: 27.06.2016
(51) Int. Cl.: C07C 263/10, C01B 7/07, C07C 265/14

(54) **VERFAHREN ZUR BEREITSTELLUNG VON CHLORWASSERSTOFF FÜR CHEMISCHE UMSETZUNGEN**
METHOD FOR PROVIDING HYDROGEN CHLORIDE FOR CHEMICAL CONVERSIONS
PROCÉDÉ DE PRÉPARATION DE CHLORURE D'HYDROGÈNE POUR RÉACTIONS CHIMIQUES

(30) Priorität: 29.06.2015 EP 15174249
(43) Veröffentlichungstag der Anmeldung: 02.05.2018
(73) Patentinhaber: Covestro Deutschland AG, 51373 Leverkusen (DE)
(72) Erfinder: BUSCH, Jan, 40477 Düsseldorf (DE); ARRAS, Jürgen, 25524 Itzehoe (DE); STEFFENS, Christian, 51067 Köln (DE)
(74) Vertreter: Levpat
(86) Internationale Anmeldenummer: PCT/EP2016/064808
(87) Internationale Veröffentlichungsnummer: WO 2017/001320

(56) Entgegenhaltungen:
- DE-B- 1 233 854

## Beschreibung

Die Erfindung betrifft ein verbessertes Verfahren zur Verfügbarmachung des bei der Herstellung eines Isocyanats durch Phosgenierung des korrespondierenden Amins anfallenden Koppelprodukts Chlorwasserstoff für eine gewünschte Folgeanwendung (d. h. eine chemische Umsetzung), bei dem ein Teil des insgesamt anfallenden Chlorwasserstoffs durch Druckerniedrigung des Rohprodukts der Phosgenierung gasförmig bei einem Druck gewonnen wird, der höher ist als der für die Folgeanwendung des Chlorwasserstoffs angestrebte Druck, und der übrige Teil des insgesamt anfallenden Chlorwasserstoffs von dem bei der Druckerniedrigung verbleibenden flüssigen Rohprodukt der Phosgenierung bei einem geringeren Druck als dem für die gewünschte Folgeanwendung angestrebten abgetrennt und anschließend auf einen Druck verdichtet wird, der höher ist als für die Folgeanwendung angestrebt, und bei dem beide so erhaltene Chlorwasserstoffströme, bevorzugt gemeinsam nach ihrer Vereinigung, so gereinigt werden, dass gereinigter Chlorwasserstoff bei einem Druck anfällt, der höher ist als der für die Folgeanwendung angestrebte Druck.

Zur Herstellung von Isocyanaten durch Phosgenierung der korrespondierenden Amine sind viele Verfahren bekannt und in der Literatur beschrieben. Abhängig vom Typ der Amine kann die Reaktion in der Gas- oder Flüssigphase sowie diskontinuierlich oder kontinuierlich durchgeführt werden (W. Siefken, Liebigs Ann. 562, 75 - 106 (1949)).

Die Durchführung kontinuierlicher Synthesen von organischen Isocyanaten im großtechnischen Maßstab ist bereits mehrfach beschrieben worden, siehe beispielsweise Ullmanns Encyklopädie der technischen Chemie, 4. Aufl. (1977), Band 13, S. 351 bis 353. Es kommen dabei sowohl aromatische Isocyanate wie beispielsweise Methylendiphenyldiisocyanat (nachfolgend MMDI - "monomeres MDI"), Polymethylen-Polyphenylen-Polyisocyanat (ein Gemisch aus MMDI und höheren Homologen, nachfolgend PMDI, "polymeres MDI") oder Toluylendiisocyanat (nachfolgend TDI) als auch aliphatische Isocyanate wie z. B. Hexamethylendiisocyanat (nachfolgend HDI) oder Isophorondiisocyanat (nachfolgend IPDI) weltweit zum Einsatz.

Nahezu ausschließlich in kontinuierlicher Fahrweise werden die industriellen Verfahren zur Produktion aromatischer Isocyanate wie MMDI, PMDI und TDI sowie aliphatischer Isocyanate wie HDI und IPDI betrieben. Als Beispiel für ein solches Verfahren in verschiedenen kontinuierlich geführten Kesseln sei DE-A-844 896 genannt.

Üblicherweise erfolgt die Phosgenierung primärer Amine (RNH₂) in Stufen, wobei zuerst bei niedriger Temperatur das Carbaminsäurechlorid (RNHCOCl) aus den Ausgangsstoffen gebildet wird und dieses anschließend bei erhöhter Temperatur in das entsprechende Isocyanat (RNCO) überführt wird, wobei in beiden Schritten Chlorwasserstoff abgespalten wird. Während der ersten Stufe, der sog. "Kaltphosgenierung", tritt als wesentliches Nebenprodukt das zu dem eingesetzten Amin korrespondierende Aminhydrochlorid ("RNH₂·HCl" = RNH₃Cl) auf, welches in der sog. "Heißphosgenierung" in Anwesenheit von Phosgen zum entsprechenden Isocyanat reagiert. Bei der Kaltphosgenierung werden üblicherweise Temperaturen unterhalb 60 °C angewendet, im Falle der Heißphosgenierung werden Temperaturen zwischen 100 °C und 200 °C erreicht. Zweistufige Verfahren werden beispielsweise in den Schriften DE-A-20 58 032, DE-A-21 53 268 und DE-A-1 233 854 erläutert.

Bei der Herstellung eines Isocyanats durch Phosgenierung des korrespondierenden Amins entstehen entsprechend der Stöchiometrie der zugrunde liegenden chemischen Reaktion erhebliche Mengen an Chlorwasserstoff. Bei großtechnischen Prozessen wird üblicherweise der Großteil des Chlorwasserstoffs direkt aus dem Rohprodukt der Reaktion durch Druckerniedrigung gasförmig gewonnen und anschließend durch Tiefkältebehandlung gereinigt ("Ausfrieren" von Verunreinigungen wie Phosgen). Das bei der Druckerniedrigung verbleibende restliche flüssige Rohprodukt wird von Phosgen befreit, beispielsweise durch Destillation, wobei neben einem flüssigen Gemisch aus Isocyanat (und i. A. Lösungsmittel) ein gasförmiges Gemisch aus Phosgen und dem restlichen Chlorwasserstoff anfällt. Hieraus wird Phosgen abgetrennt, beispielsweise durch Absorption, wodurch der restliche Chlorwasserstoff gasförmig gewonnen wird und anschließend mit der Hauptmenge an Chlorwasserstoff vereinigt werden kann. Diese Verfahrensführung ist grundsätzlich beschrieben in SRI International, Report No. 1C, ISOCYANATES PART I, SUPPLEMENT C, Yen-Chen Yen, July 1979. So gewonnener Chlorwasserstoff verlässt den Prozess in vielen Verfahren bei einem Druck, der geringer als für Folgeanwendungen angestrebt ist, und enthält darüber hinaus immer noch verschiedene Verunreinigungen, wie beispielsweise Reste von Phosgen, Lösungsmitteln, organischen Nebenprodukten und Inertgasen, welche für Folgeanwendungen u. U. schädlich sein können.

Um ein Verfahren zur Herstellung von Isocyanaten in großtechnischem Maßstab wirtschaftlich betreiben zu können, ist es unbedingt erforderlich, den anfallenden Chlorwasserstoff einer wirtschaftlich sinnvollen Verwendung zuzuführen. Dies kann nach dem Stand der Technik auf verschiedene Weisen geschehen; die wichtigsten sind die Erzeugung von Salzsäure (typischerweise einer Konzentration von oberhalb 30 %) durch Absorption in Wasser und die katalytische Oxidation des Chlorwasserstoffs mit Sauerstoff zu Chlor (sog. Deacon-Prozess). Im ersteren Fall kann die Salzsäure entweder direkt einer geeigneten Verwendung zugeführt oder elektrolytisch zu Chlor oxidiert werden. Aus dem Koppelprodukt Chlorwasserstoff einer Isocyanat-Produktion gewonnenes Chlor kann (unabhängig davon, ob es durch katalytische Oxidation des Chlorwasserstoffs oder Elektrolyse zuvor aus dem Chlorwasserstoff hergestellter Salzsäure gewonnen wurde) mit Kohlenstoffmonoxid zu Phosgen, einem der Ausgangsstoffe der Isocyanat-Produktion, umgesetzt werden, sodass es möglich ist, eine Isocyanat-Produktion mit einem im Wesentlichen geschlossenen Chlorkreislauf zu betreiben. Daneben ist aber auch möglich, den anfallenden Chlorwasserstoff einer Verwendung außerhalb der Isocyanat-Produktion zuzuführen. Beispielhaft sei die Verwendung von aus dem Chlorwasserstoff gewonnenem Chlor zur Herstellung von Ethylendichorid (1,2-Dichlorethan, nachfolgend EDC) genannt, einem wichtigen Ausgangsstoff beispielsweise für die Herstellung von Polyvinylchlorid und Ethylendiamin.

Abhängig von der erwünschten Art der Weiterverwendung des Chlorwasserstoffs werden an dessen Reinheit und Druck unterschiedliche Anforderungen gestellt. Die Absorption mit Wasser zur Herstellung von Salzsäure ist etwa bzgl. des Drucks und der Reinheit vergleichsweise anspruchslos. Demgegenüber muss Chlorwasserstoff, der in einem Deacon-Verfahren eingesetzt werden soll, einen hohen Reinheitsgrad im Hinblick auf organische Verunreinigungen und einen erhöhten Druck von bis zu 25 bar (absolut) aufweisen. Verfahren zur Herstellung von EDC erfordern einen Eingangsdruck des Chlorwasserstoffs von bis zu 15 bar (absolut) und stellen ebenfalls erhöhte Anforderungen an dessen Reinheit.

Da, wie bereits erwähnt, bei vielen Isocyanat-Produktionsverfahren der Chlorwasserstoff bei einem Druck anfällt, der geringer ist als für viele Folgeanwendungen angestrebt, muss dieser daher für die Mehrzahl der Folgeanwendungen nicht nur gereinigt, sondern auch verdichtet werden. Dies ist mit erhöhtem apparativem und energetischem Aufwand verbunden. Es gibt jedoch auch Isocyanat-Produktionsverfahren, bei denen zumindest ein Teil des Chlorwasserstoffs, je nach Ausgestaltung der gesamte Chlorwasserstoff, bei einem deutlich höheren Druck anfällt. So wird in EP 1 616 857 A1 ein Verfahren beschrieben, in dem das Rohprodukt der Reaktion bei einem Druck von bis zu 15 bar anfällt, es mithin möglich ist, auch den Chlorwasserstoff bei einem relativ hohen Druck zu gewinnen, sodass zumindest der Verfahrensschritt der Verdichtung des Chlorwasserstoffs grundsätzlich entfallen könnte. Weitere Beispiele für Verfahren, bei denen es grundsätzlich möglich ist, den als Koppelprodukt anfallenden Chlorwasserstoff bei erhöhtem, für Folgeanwendungen ausreichendem Druck zu gewinnen, sind in der noch nicht veröffentlichten europäischen Patentanmeldung Nr. EP15174217.8 beschrieben. Wenn der Chlorwasserstoff auch nicht für alle Folgeanwendungen im Hinblick auf organische Verunreinigungen extrem rein sein muss, so muss er doch in jedem Fall weitest möglich von überschüssigem Phosgen befreit werden.

Die Abtrennung von Phosgen und Chlorwasserstoff und darin enthaltenen Resten von Lösungsmittel, Isocyanat, weiteren organischen Komponenten, Inertgasen etc. aus dem Rohprodukt der Phosgenierung und die anschließende Trennung von Chlorwasserstoff und die Rückführung von Phosgen werden in vielen Schriften beschrieben (z. B. EP1849767B1, EP1575906B1, WO2009/059903A1, WO2005/115974A1, EP1575904B1, US7,592,479B2, DE2252068, EP2093215A1, EP1401802B1, EP1529033B1, EP2200976B1, WO2011/003532A1, WO2009/037179A1, WO2013/026591A1).

In EP 1 849 767 B1 wird die Trennung von Phosgen und Chlorwasserstoff in einem Rieselfilmabsorber realisiert. Der Absorber arbeitet bevorzugt bei 1 bis 35 bar, besonders bevorzugt bei 1,2 bis 3 bar. Der Zweck des Rieselfilmabsorbers ist vorrangig die Erzeugung eines möglichst reinen Chlorwasserstoff-Gases (über Kopf), das einer Weiterverarbeitung oder einer Chlorrückgewinnung zugeführt wird. Der Vorteil eines Rieselfilmabsorbers liegt darin, dass das Phosgen im aufgegebenen Lösungsmittel absorbiert und damit abgetrennt werden kann, ohne dass der Chlorwasserstoff selbst als Rücklauf verwendet und damit kondensiert werden muss. Nachteilig an der Verwendung eines Rieselfilmabsorbers sind die vergleichsweise hohen Apparatekosten und die für manche Anwendungen nicht ausreichende Reinheit des Chlorwasserstoffs.

EP 2 021 275 B1 beschreibt die weitergehende destillative Reinigung eines Chlorwasserstoffstroms, wie er z. B. aus dem zuvor erwähnten Rieselfilmabsorber gewonnen wird. Das Gas wird auf einen Druck zwischen 5 und 30 bar komprimiert, teils kondensiert und in einer Destillationskolonne aufgetrennt. Am Kopf wird dabei hochreiner Chlorwasserstoff gewonnen, am Sumpf werden Reste von Phosgen, Lösungsmittel, Organika etc. entnommen. Eine Energieintegration minimiert die eingesetzte Kühlenergie. Vorteilhaft bei dem Verfahren sind die hohe Reinheit des Chlorwasserstoffs sowie der geringe Energieverbrauch zur Destillation. Nachteilig ist, dass der gesamte zu reinigende Gasstrom mit entsprechendem Energieeinsatz komprimiert werden muss. Das beanspruchte Verfahren hat zudem den wirtschaftlichen Nachteil, dass anfallende Sumpfströme als Reststoff entsorgt werden.

EP 1 575 906 B1 beschreibt die Trennung von Chlorwasserstoff und Phosgen bei einem bevorzugten Druck von 3 bis 16 bar. Dabei wird das Gasgemisch zunächst partiell kondensiert und dann destilliert oder gestrippt, wobei das Sumpfprodukt kaum noch Chlorwasserstoff enthält. Das Kopfprodukt wird einem Wäscher zugeführt, um verbleibendes Phosgen aus dem Chlorwasserstoff zu entfernen. Der so erhaltene Chlorwasserstoff kann danach komprimiert und einem Deacon- oder EDC-Verfahren zugeführt werden. Nachteilig sind hier die Zahl der Prozessschritte und damit Apparate sowie die notwendige Komprimierung des gesamten Chlorwasserstoffes.

In US 6,719,957 B2 wird ein Verfahren zur Reinigung eines Gases von Verunreinigungen beschrieben, deren Siedepunkt größer als 100 °C ist. Es beinhaltet eine Komprimierung des Gases von 1 bis 5 bar auf 8 bis 20 bar und eine nachfolgende 2-stufige Kondensation zur Entfernung von Verunreinigungen. Dabei wird eine Energieintegration zwischen den beiden Stufen realisiert.

Nachteilig bei dem Verfahren ist der Umstand, dass Phosgen und andere Komponenten mit Siedepunkten kleiner als 100 °C nicht abgetrennt werden.

WO 2009/059903 A1 beschreibt die destillative Trennung von Reaktionsprodukt, Phosgen und Chlorwasserstoff bei Drücken von 3 über 10 bis 30 bar. Das Reaktionsprodukt wird flüssig am Sumpf der Kolonne abgezogen, Phosgen wird ebenfalls flüssig in einem Seitenabzug entnommen, und Chlorwasserstoff wird gasförmig am Kopf abgezogen. Da der durch Kondensatoren erzeugte Rücklauf auf die Kolonne aus Phosgen und Chlorwasserstoff bestehen soll, enthält der die Kolonne verlassene Chlorwasserstoff noch merkliche Mengen an Phosgen. Nachteilig bei niedrigen Betriebsdrücken unterhalb des für eine Folgeanwendung benötigten Chlorwasserstoff-Abgabedrucks ist außerdem, dass eine weitere Komprimierung des gesamten Chlorwasserstoff-Stroms notwendig ist. Bei alternativ hohem Betriebsdruck ist nachteilig, dass entsprechend hohe Mengen an niedrig siedendem Lösungsmittel oder Phosgen benötigt werden, um die Sumpftemperatur in der Kolonne zu begrenzen. Letztes ist notwendig, um ungewünschte Folgereaktionen (Farbentwicklung, Polymerisation) zu vermeiden. Der Einsatz hoher Mengen von Lösungsmittel oder Phosgen bedingt aber hohen Aufwand und Kosten bei der Aufarbeitung.

In DE 1593412 wird die Herstellung reinen Chlorwasserstoffs als Nebenprodukt der Isocyanatherstellung bei über 10 atü beschrieben. Hierbei wird der gesamte freie und gebundene Chlorwasserstoff in einer ersten Destillationskolonne direkt nach der Phosgenierung abgetrennt. Zur Minimierung der Phosgenverluste kann dieser Chlorwasserstoff in einer weiteren Kolonne von Phosgen befreit werden. Merkmal des Verfahrens ist die vollständige Umsetzung des Zwischenprodukts Carbamylchlorid, das noch gebundenen Chlorwasserstoff enthält, zum Isocyanat am Sumpf der ersten Kolonne, so dass kein Chlorwasserstoff in der weiteren Aufarbeitung des Isocyanats abgetrennt werden muss. Nachteilig ist wiederum, dass bei höherem Druck in der Kolonne entsprechend hohe Mengen an niedrig siedendem Lösungsmittel oder Phosgen benötigt werden, um die Sumpftemperatur zu begrenzen.

WO 2005/115974 A1 beschreibt ein Verfahren zur Herstellung von Isocyanaten, bei dem der aus dem Reaktionsgemisch abgetrennte Gasstrom, der im Wesentlichen Phosgen, Chlorwasserstoff und Lösungsmittel enthält, in einer Trenneinheit, zum Beispiel einer Destillationskolonne aufgetrennt wird. Erfindungswesentlich ist die Reinheit des rückgewonnenen Phosgens bzgl. Chlorwasserstoff und Isocyanaten. Die Reinheit des Chlorwasserstoffs wird jedoch nicht betrachtet.

WO 2013/026592 beschreibt die Trennung von Chlorwasserstoff und Phosgen in einer Kolonne, bei der am Kopf eine flüssige Mischung enthaltend Chlorwasserstoff und Phosgen aufgegeben wird und am Sumpf eine gasförmige Mischung der beiden Komponenten und optional eines Strippgases, z. B. Stickstoff oder Kohlenstoffmonoxid, eingebracht wird. Im oberen Teil der Kolonne wird Phosgen aus dem Chlorwasserstoff gewaschen, während im unteren Teil Chlorwasserstoff aus dem Phosgen gestrippt wird. Nachteilig ist, dass am Kopf aufgegebenes Phosgen als Verunreinigung im am Kopf abgetrennten Chlorwasserstoff auftreten wird, ebenso wie eingesetzter Stickstoff oder Kohlenstoffmonoxid.

Die Entfernung von (Chlor-)Kohlenwasserstoffen aus Chlorwasserstoff mittels Adsorption wird in WO 2007/085627 A1 beschrieben, etwa um den Chlorwasserstoff einem Deacon-Prozess zuzuführen. Das Verfahren zielt darauf ab, die entfernten (Chlor-)Kohlenwasserstoffe Phosgen-frei zurück zu gewinnen. Dies bedingt jedoch, dass das Phosgen nicht aus dem Chlorwasserstoff entfernt wird. Nachteilig ist also, dass die Entfernung des Phosgens zusätzlich bereits zuvor oder danach erfolgen muss, beispielsweise durch Absorption, was wiederum die Zahl der Prozessschritte und Kosten erhöht.

In WO2013/026591 A1 werden Membranen eingesetzt, um ein Gemisch aus Phosgen und Chlorwasserstoff aufzutrennen, um einen Phosgen-reichen und Chlorwasserstoffen-armen sowie einen Chlorwasserstoff-reichen und einen Phosgen-armen Strom zu erhalten. Nachteilig sind die relativ hohen Anschaffungs- und Betriebskosten für Membrantrenneinheiten.

Zusammenfassend lässt sich feststellen, dass die beschriebenen Verfahren des Standes der Technik im Allgemeinen zwar verlässlich betrieben werden können, jedoch nicht frei von Nachteilen sind. Die zur Bereitstellung des bei einer Isocyanatherstellung anfallenden Koppelprodukts Chlorwasserstoff für Folgeanwendungen, insbesondere solche Folgeanwendungen, die hinsichtlich des angestrebten Chlorwasserstoffdrucks und / oder der angestrebten Chlorwasserstoffreinheit besondere Anforderungen stellen, ist mit relativ hohem apparativem und verfahrenstechnischem Aufwand verbunden, sodass hier noch Verbesserungsbedarf besteht.

Dem Vorstehend Gesagten Rechnung tragend ist ein Gegenstand der vorliegenden Erfindung ein Verfahren zur Verfügbarmachung von bei der Herstellung eines Isocyanats durch Phosgenierung des korrespondierenden Amins (10), ggf. in Gegenwart eines Lösungsmittels (30), als Koppelprodukt anfallenden Chlorwasserstoffs für eine gewünschte Folgeanwendung (d. h. für eine chemische Umsetzung) mit einem angestrebten Eingangsdruck des dort einzusetzenden Chlorwasserstoffs (100) von p_{F}, umfassend die Schritte:
(i) ein- oder mehrstufige Entspannung des Rohprodukts der Phosgenierung (40), das unter dem Druck p_{R} steht, auf einen Druck p_{E} ≥ p_{F}, wodurch ein gasförmiger, überwiegend Chlorwasserstoff enthaltender Strom (60) und ein flüssiger, überwiegend Isocyanat- und Phosgen sowie ggf. Lösungsmittel enthaltender Strom (50) gewonnen werden;
(ii) Abtrennung von Phosgen aus dem überwiegend Isocyanat- und Phosgen sowie ggf. Lösungsmittel enthaltenden Strom (50) bei einem Druck p_{A} < p_{F}, wobei ein gasförmiger, Phosgen- und Chlorwasserstoff sowie ggf. eine untergeordnete Menge des Lösungsmittels enthaltender Strom (80) und ein Isocyanat sowie ggf. die Hauptmenge des Lösungsmittels enthaltender Strom (70) erhalten werden;
(iii) Verdichtung des Phosgen- und Chlorwasserstoff sowie ggf. eine untergeordnete Menge des Lösungsmittels enthaltenden Stroms (80) auf einen Druck p_{V} > p_{F}, wobei ein verdichteter Gasstrom (90) erhalten wird;
(iv) Reinigung, und zwar bevorzugt in einer Destillationskolonne, des Chlorwasserstoff enthaltenden Stroms (60) und des verdichteten Phosgen- und Chlorwasserstoff sowie ggf. eine untergeordnete Menge des Lösungsmittels (30) enthaltenden Stroms (90), bevorzugt nach Vereinigung der beiden Ströme, bei einem Druck p_{D} > p_{F}, wobei gereinigtes Chlorwasserstoffgas (100) und Phosgen-haltige Flüssigkeit (110) erhalten werden.

Das erfindungsgemäße Verfahren eignet sich besonders für die Anwendung in der Herstellung von Methylendiphenyldiisocyanat (MMDI) als reine Isomere oder als Isomerengemisch, Polymethylenpolyphenylpolyisocyanat (PMDI - höhere Homologe von MMDI mit drei oder mehr Benzolkernen), Gemischen aus Methylendiphenyldiisocyanat und Polymethylenpolyphenylpolyisocyanat, Toluylendiisocyanat (TDI) als reine Isomere oder Isomerengemisch, Isomeren des Xylylendiisocyanats (XDI), Isomeren des Diisocyanatobenzols, 2,6-Xylolisocyanat, 1,5-Naphthylendiisocyanat (1,5-NDI), Diisocyanaten auf Basis von aliphatischen oder cycloaliphatischen Kohlenwasserstoffen mit 2 bis 18 Kohlenstoffatomen, wie z. B. 1,4-Butandiisocyanat, 1,5-Pentandiisocyanat, 1,6-Hexandiisocyanat (HDI), 1,8-Octandiisocyanat, 1,9-Nonandiisocyanat, 1,10-Decandiisocyanat, 2,2-Dimethylpentan-1,5-diisocyanat, 2-Methyl-1,5-pentandiisocyanat (MPDI), 2,4,4(oder 2,2,4)-Trimethyl-1,6-hexandiisocyanat (TMDI), 1,3- und 1,4-Cyclohexandiisocyanat, 1-Isocyanato-3,3,5-trimethyl-5-isocyanatomethylcyclohexan (IPDI), 2,4-, oder 2,6-Diisocyanato-1-methylcyclohexan (H6-TDI), 1-Isocyanato-1-methyl-4(3)-isocyanatomethylcyclohexan (AMCI), 1,3(und/oder 1,4)-Bis(isocyanatomethyl)cyclohexan, Bis(isocyanatomethyl)norbornan (NBDI), 4,4'(und/oder 2,4')-Diisocyanatodicyclohexyl-methan, und (cyclo)aliphatische Triisocyanate mit bis zu 22 Kohlenstoffatomen, wie z.B. Triisocyanatocyclohexan, Tris(isocyanatomethy1)-cyclohexan, Triisocyanato-methylcyclohexan, 1,8-Diisocyanato-4-(isocyanatomethyl)octan, 1,6,11-Undecantriisocyanat, 1,7-Diisocyanato-4-(3-isocyanatopropyl)heptan, 1,6-Diisocyanato-3-(isocyanatomethyl)-hexan oder 1,3,5-Tris(isocyanatomethy1)-cyclohexan. Ganz besonders bevorzugt eignet sich das erfindungsgemäße Verfahren für die Anwendung in der Herstellung von Toluylendiisocyanat, Diphenylmethandiisocyanat und Polyphenylenpolymethylenpolyisocyanat.

Die korrespondierenden Amine zu den obigen Polyisocyanaten sind aromatische Polyamine, wie z. B. Methylendiphenyldiamin (MMDA) als reine Isomere oder als Isomerengemisch, Polymethylenpolyphenylpolyamin (PMDA), Gemische aus Methylendiphenyldiamin und Polymethylenpolyphenylpolyamin, Toluylendiamin (TDA) als reine Isomere oder Isomerengemisch, Isomere des Xylylendiamins (XDA), Isomere des Diaminobenzols, 2,6-Xylidin, 1,5-Naphthylendiamin (1,5-NDA), Polyamine auf Basis von aliphatischen oder cycloaliphatischen Kohlenwasserstoffen mit 2 bis 18 Kohlenstoffatomen, wie z.B. 1,4-Diaminobutan, 1,5-Diaminopentan, 1,6-Diaminohexan (HDA), 1,8-Diaminooctan, 1,9-Diaminononan, 1,10-Diaminodecan, 2,2-Dimethyl-1,5-diaminopentan, 2-Methyl-1,5-pentandiamin (MPDA), 2,4,4(oder -2,2,4)-Trimethyl-1,6-diaminohexan (TMDA), 1,3- und 1,4-Diaminocyclohexan, 1-Amino-3,3,5-trimethyl-5-aminomethylcyclohexan (IPDA), 2,4-, oder 2,6-Diamino-1-methylcyclohexan (H6-TDA), 1-Amino-1-methyl-4(3)-aminomethylcyclohexan (AMCA), 1,3(und/oder 1,4)-Bis(aminomethyl)cyclohexan, Bis(aminomethyl)norbornan (NBDA), 4,4'(und/oder 2,4')-Diaminodicyclohexyl-methan, (cyclo)aliphatische Polyamine mit bis zu 22 Kohlenstoffatomen, wie z. B. Triaminocyclohexan, Tris(aminomethyl)-cyclohexan, Triamino-methylcyclohexan, 1,8-Diamino-4-(aminomethyl)octan, 1,6,11-Undecantriamin, 1,7-Diamino-4-(3-aminopropyl)heptan, 1,6-Diamino-3-(aminomethyl)-hexan oder 1,3,5-Tris(aminomethyl)-cyclohexan.

Das erfindungsgemäße Verfahren eignet sich besonders für die Anwendung in der Herstellung von Methylendiphenyldiisocyanat (MMDI) als reine Isomere oder als Isomerengemisch, Polymethylenpolyphenylpolyisocyanat (PMDI), Gemische aus Methylendiphenyldiisocyanat und Polymethylenpolyphenylpolyisocyanat, Toluylendiisocyanat (TDI) als reine Isomere oder Isomerengemisch, Isomere des Xylylendiisocyanats (XDI), Isomere des Diisocyanatobenzols, 2,6-Xylolisocyanat, 1,5-Naphthylendiisocyanat (1,5-NDI).

Besonders bevorzugt ist die Anwendung des erfindungsgemäßen Verfahrens in der Herstellung von Diphenylmethandiisocyanat (MMDI) und/oder Polyphenylenpolymethylenpolyisocyanat (PMDI). MMDI und PMDI werden im Rahmen dieser Erfindung auch, unabhängig von Polymerisationsgrad und Isomerenverteilung, summarisch als MDI bezeichnet; Analoges gilt für MDA.

Die Herstellung der korrespondierenden Polyamine ist aus dem Stand der Technik hinreichend bekannt und wird daher an dieser Stelle nicht im Detail ausgeführt. Im Fall des besonders bevorzugten Polyisocyanats MDI wird das korrespondierende Polyamin MDA durch säurekatalysierte Kondensation von Anilin und Formaldehyd erhalten. Dabei entsteht ein Gemisch aus der "Zweikernverbindung" MMDA (enthaltend zwei Benzolringe mit je einer Aminogruppe) und höheren Homologen PMDA ("Mehrkernverbindungen", enthaltend drei oder mehr Benzolringe mit je einer Aminogruppe). Bei den meisten großtechnisch üblichen Verfahren wird dieses Gemisch ohne vorherige Trennung in monomere und polymere Bestandteile phosgeniert. Eine Trennung in monomere Bestandteile und polymere Bestandteile findet daher meist erst auf der Stufe des Polyisocyanats statt. Dabei werden einerseits die Zweikemverbindung (MMDI) und andererseits ein Gemisch aus der Zweikernverbindung (MMDI) und den höheren Homologen (PMDI) erhalten.

Nachstehend werden Ausführungsformen der Erfindung näher beschrieben. Dabei sind verschiedene Ausführungsformen beliebig miteinander kombinierbar, solange sich für den Fachmann aus dem Kontext nicht eindeutig das Gegenteil ergibt.

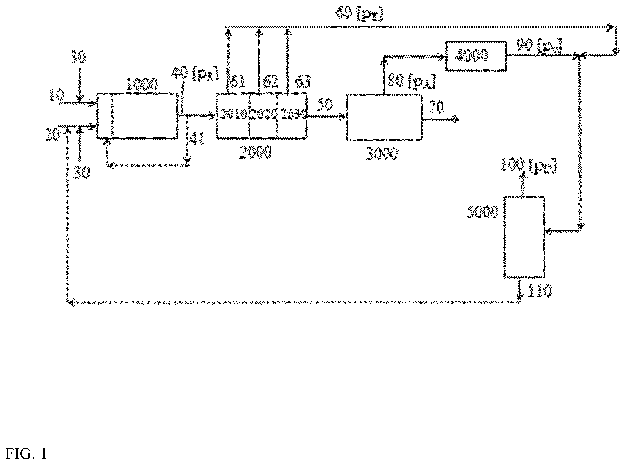

Eine mögliche Ausgestaltung des erfindungsgemäßen Verfahrens, in welcher auch die Phosgenierungsreaktion gezeigt ist, ist in FIG. 1 dargestellt:
Amin (10) und Phosgen (20) werden in einem unter den Reaktionsbedingungen inerten Lösungsmittel (30) gelöst, und zwar bevorzugt so, dass
- der resultierende Phosgen-haltige Strom einen Massenanteil von 5 % bis 95 %, bevorzugt von 20 % bis 75 %, besonders bevorzugt von 30 % bis 60 %, an Phosgen und einen Massenanteil von 95 % bis 5 %, bevorzugt von 80 % bis 25 %, besonders bevorzugt von 70 % bis 40 %, eines inerten Lösungsmittels aufweist, jeweils bezogen auf die Gesamtmasse des Phosgen-haltigen Stroms,
- der Amin-haltige Strom einen Massenanteil von 5 % bis 95 %, bevorzugt von 10 % bis 60 %, besonders bevorzugt von 30 % bis 45 %, an Amin und einen Massenanteil von 95 % bis 5 %, bevorzugt von 90 % bis 40 %, besonders bevorzugt von 75 % bis 55 %, eines inerten Lösungsmittels aufweist, jeweils bezogen auf die Gesamtmasse des Amin-haltigen Stroms.

*Unter den Reaktionsbedingungen inert* bedeutet dabei, dass das Lösungsmittel nicht in signifikantem Umfang mit den Edukten, Zwischenprodukten und Endprodukten der Reaktion reagiert. Daher wird bevorzugt das inerte Lösungsmittel (30) für Strom (10) und Strom (20) unabhängig voneinander ausgewählt aus Chlorbenzol, Dichlorbenzol, Toluol, Dioxan, Dimethylsulfoxid oder einer Mischung aus zwei oder mehr der vorgenannten Lösungsmittel.

Besonders bevorzugt für beide Ströme (10) und (20) sind Chlorbenzol und Dichlorbenzol, ganz besonders bevorzugt ist Chlorbenzol. Im Fall des Dichlorbenzols ist das ortho-Isomer (ortho-Dichlorbenzol) besonders bevorzugt.

Die so erhaltenen Amin- und Phosgen-haltigen Ströme werden vermischt (nicht gezeigt in FIG. 1) und in einem Reaktor (1000) umgesetzt, wobei die eingesetzte Mischapparatur auch in den Reaktor (1000) integriert sein kann. Dabei kann eine beliebige aus dem Stand der Technik bekannte Mischapparatur eingesetzt werden. Exemplarisch seien *statische Mischer*, und hier insbesondere Düsen, wie beispielsweise Ringschlitzdüsen (DE-A-1792660), Ringlochdüsen (DE-C1-3744001), Glattstrahlmischdüsen (EP-A1-0 065 727), Fächerstrahldüsen (DE-A1-2950216), Winkelstrahlkammerdüsen (DD-A7-300.168), Dreistromdüsen (DD-A1-132340), Gegenstrommischkammern (DE-B1146872), Staudüsen (FR-E-69428) und Venturimischdüsen (DE-B1175666) genannt. Ferner eignen sich *dynamische Mischer*, insbesondere Rotor-Stator- oder turbinenartige Systeme, bei denen die Reaktanden im Gleichstrom in die Mischeinheit eingeleitet werden. Als Beispiel kann der in EP-A-2 077 150 beschriebene Mischer genannt werden.

Bei der Umsetzung im Reaktor (1000) fällt ein Rohprodukt (40) an, das Isocyanat, Lösungsmittel, Chlorwasserstoff, nicht umgesetztes Phosgen sowie, abhängig von den genauen Reaktionsbedingungen, Anteile an Carbaminsäurechlorid und ggf. kleinere Mengen Aminhydrochlorid umfasst. Voraussetzung für die Durchführung des erfindungsgemäßen Verfahrens ist lediglich, dass dieses Rohprodukt bei einem Druck p_{R} anfällt, der größer ist als der für die Folgeanwendung des Chlorwasserstoffs angestrebte Druck p_{F}, und zwar mindestens so viel größer, dass der in Schritt (i) (siehe unten für Details) anfallende Gasstrom (60), der im Gegensatz zum in Schritt (ii) anfallenden Gasstrom (80) nicht mehr verdichtet wird, ein Druckniveau aufweist, das eine ausreichende Gasströmung über die in Schritt (iv) einzusetzende Reinigungsapparatur (5000) zur Folgeanwendung hin ermöglicht. Solange diese Voraussetzung erfüllt ist, kann die Umsetzung im Reaktor (1000) nach einem beliebigen bekannten Verfahren des Standes der Technik durchgeführt werden. Bevorzugt beträgt p_{R} 6,00 bar bis 60,0 bar, besonders bevorzugt 12,0 bar bis 45,0 bar. Das Rohprodukt (40) weist bevorzugt eine Temperatur T_{R} im Bereich von 80 °C bis 200 °C, bevorzugt von 110 °C bis 170 °C auf.

So kann als Reaktor (1000) grundsätzlich jeder aus dem Stand der Technik bekannte Phosgenierreaktor eingesetzt werden. Bevorzugt werden senkrecht stehende und von unten durchströmte Rohrreaktoren verwendet. Zur Einengung der Verweilzeit kann der Rohrreaktor durch geeignete, dem Fachmann bekannte Einbauten segmentiert sein. Von der vorliegenden Erfindung mit umfasst sind auch Ausführungsformen, bei denen mehrere Reaktoren (1000) in Reihe oder parallel geschaltet sind.

In einer Ausführungsform des erfindungsgemäßen Verfahrens wird der Reaktor (1000) adiabat, d. h. ohne gezielte Wärmezu oder -abfuhr, betrieben. In einem solchen Verfahren spiegelt sich die Reaktionsenthalpie - unter Vernachlässigung unvermeidbarer Wärmeverluste - quantitativ in der Temperaturdifferenz zwischen Ausgangs- und Eingangsstrom wider. Zur Vermeidung von Wärmeverlusten wird der Reaktor bevorzugt isoliert. In der Druckschrift EP 1 616 857 A1 ist die adiabate Betriebsweise bei der Polyaminphosgenierung näher beschrieben, insbesondere in den Absätzen [0014] bis [0018].

In einer anderen Ausführungsform des erfindungsgemäßen Verfahrens wird der Reaktor (1000) isotherm, d. h. unter Zufuhr von Wärme über einen thermostatisierbaren Reaktor durch ein geeignetes Wärmeträgermedium (z. B. Wärmeträgeröl, Salzschmelze), betrieben. Beispielhaft seien die Schriften DE 1768439 A1, insbesondere Absatz [0003] auf Seite 8 und EP 1 616 857 B1, insbesondere die Absätze [0021] bis [0022], für die Phosgenierung von Polyaminen unter isothermer Betriebsweise genannt.

Es ist auch möglich (in FIG. 1 nicht gezeigt), eine Sequenz mehrerer Reaktoren (1000) einzusetzen, die unabhängig voneinander isotherm oder adiabat betrieben werden können. Bevorzugt ist eine Sequenz aus zwei hintereinandergeschalteten Reaktoren, von denen der erste adiabat und der zweite isotherm betrieben wird, wie in EP 1 616 857 A1 beschrieben.

Es ist ferner möglich, aus dem Rohprodukt (40) einen Teilstrom (41) abzuzweigen und in die Vermischung von Amin- und Phosgen-haltigem Strom zurückzuführen ("Reaktionskreislauf"; in FIG. 1 gestrichelt gezeichnet). Ein solches Verfahren wird in der noch nicht veröffentlichten europäischen Patentanmeldung Nr. EP15174217.8 beschrieben.

Das unter dem Druck p_{R} anfallende Rohprodukt (40) wird in einem Abscheider (2000) durch Entspannung, also unter Druckerniedrigung auf einen Druck p_{E} > p_{F}, in eine flüssige Phase (50), die überwiegend Isocyanat- und Phosgen sowie Lösungsmittel (30) enthält, und eine gasförmige Phase (60), die überwiegend Chlorwasserstoff sowie untergeordnete Anteile an Phosgen enthält, getrennt (**Schritt (i)**). Der Abscheider (2000) kann auch als eine Kaskade mehrerer hintereinander geschalteter Abscheider (2010, 2020, 2030, ...) mit sukzessive sinkendem Druckniveau ausgestaltet sein, wobei die in einem Abscheider erhaltene Flüssigphase in den folgenden Abscheider geleitet wird (mit Ausnahme natürlich der flüssigen Phase des letzten Abscheiders, welche den überwiegend Isocyanat- und Phosgen sowie ggf. Lösungsmittel enthaltenden Strom (50) darstellt). Die einzelnen Gasphasen (61, 62, 63, ...) werden zum Gasstrom (60) vereinigt, der einen Druck p_{E} aufweist und bevorzugt 30 % bis 80 %, besonders bevorzugt 50 bis 70 %, des insgesamt anfallenden Chlorwasserstoffs umfasst. Abscheider (2000) und Reaktor (1000) können auch in einer einzigen Apparatur vereint sein. Es ist auch möglich (in FIG. 1 nicht gezeigt), schon während der eigentlichen Umsetzung von Amin und Phosgen einen gasförmigen Purge-Strom aus dem Reaktor (1000) auszuschleusen. Ein solcher, überwiegend Chlorwasserstoff enthaltender Purge-Strom wird mit den übrigen Strömen (61, 62, 63, ...) zum Gasstrom (60) vereint. Bevorzugt beträgt p_{E} 5,00 bar bis 30,0 bar, besonders bevorzugt 9,00 bar bis 18,0 bar. Der Gasstrom (60) weist bevorzugt eine Temperatur T_{E} im Bereich von 90 °C bis 170 °C, bevorzugt von 110 °C bis 150 °C auf..

Die den letzten Abscheider verlassende Flüssigphase (50) wird in der Apparatur (3000) bei einem Druck p_{A} < p_{F} von Phosgen befreit, wobei ein gasförmiger, Phosgen- und Chlorwasserstoff sowie eine untergeordnete Menge des Lösungsmittels enthaltender Strom (80) und ein Isocyanat sowie die Hauptmenge des Lösungsmittels enthaltender Strom (70) erhalten werden (**Schritt (ii)**). In einer bevorzugten Ausführungsform ist die Apparatur (3000) eine Destillationskolonne, im Folgenden als Entphosgenierkolonne bezeichnet. Der Druck p_{A} bezeichnet in diesem Fall den Druck am Kopf der Entphosgenierkolonne und beträgt bevorzugt von 0,50 bar bis 5,00 bar, besonders bevorzugt 1,00 bar bis 3,00 bar. Das die Entphosgenierkolonne (3000) verlassende gasförmige Produkt (80) weist bevorzugt eine Temperatur T_{A} im Bereich von 10 °C bis 90 °C, bevorzugt von 30 °C bis 70 °C auf. Die Entphosgenierkolonne (3000) ist bevorzugt als Glockenbodenkolonne ausgeführt. Es können auch mehrere Destillationskolonnen hintereinandergeschaltet werden. In diesem Fall bezeichnet p_{A} den Druck am Kopf der letzten Destillationskolonne.

Der in der Entphosgenierkolonne erhaltene flüssige Strom (70) wird gemäß dem Stand der Technik von Lösungsmittel (30) befreit und weiter aufgearbeitet (in FIG. 1 nicht gezeigt).

Im Unterschied zum Stand der Technik wird die in der Entphosgenierkolonne (3000) gewonnene Gasphase (80), die bevorzugt 20 % bis 70 %, besonders bevorzugt 30 % bis 50 % des insgesamt anfallenden Chlorwasserstoffs umfasst, nicht einer weiteren Stufe zur Abtrennung von Phosgen (z. B. durch Absorption in einem Rieselfilmabsorber) zugeführt, sondern ohne weitere Reinigung in einem Verdichter (4000) auf den Druck p_{V} (Strom (90)) verdichtet (**Schritt (iii)**). Geeignete Verdichter für die Verdichtung korrosiver Gase sind dem Fachmann bekannt und werden beispielsweise in EP 2 021 275 B1 beschrieben. Beispielhaft seien Turboverdichter, Kolbenverdichter, Schraubenverdichter oder Flüssigkeitsringverdichter genannt. Bevorzugt beträgt pv 5,00 bar bis 30,0 bar, besonders bevorzugt 9,00 bar bis 18,0 bar. Strom (90) weist bevorzugt eine Temperatur T_{V} im Bereich von 90 °C bis 170 °C, bevorzugt von 110 °C bis 150 °C auf. Es ist besonders bevorzugt, wie in FIG. 1 gezeigt, den Strom (90) mit dem Gasstrom (60) zu vereinigen. Sofern vorhanden, sollte die Druckdifferenz zwischen p_{E} und p_{V} so gewählt werden, dass diese am Punkt der Vereinigung der beiden Ströme vernachlässigbar gering ist. Hierbei sind, wie dem Fachmann bekannt ist, Druckverluste infolge von in Rohrleitungen zu überwindenden Entfernungen zu berücksichtigen, d. h. p_{E} und p_{V} sollten so gewählt werden, dass eine ausreichende Strömung der entsprechenden Gasströme zum Vereinigungspunkt und von da zur Reinigungsapparatur (5000) gewährleistet ist. Es ist auch denkbar, dass p_{E} und p_{V} gleich sind. Im Falle einer Druckdifferenz zwischen den beiden Drücken beträgt diese bevorzugt > 0,00 bar bis 7,00 bar, besonders bevorzugt 1,00 bar bis 5,00 bar.

Die Reinigungsapparatur (5000) zur Durchführung von **Schritt (iv)** ist bevorzugt eine Destillationskolonne. Der Druck p_{D} entspricht dann dem Druck am Kopf der Destillationskolonne, wo der gasförmige Strom gereinigten Chlorwasserstoffs (100) entnommen wird. Dem Sumpf der Destillationskolonne wird eine Phosgen-haltige Flüssigphase (110) entnommen, welche bevorzugt wieder in die Reaktion zurückgeführt wird (in FIG. 1 gestrichelt gezeichnet). Bevorzugt wird die Flüssigphase (110) mit dem Phosgen-haltigen Strom aus Phosgen (20) und Lösungsmittel (30) vermischt. Bevorzugt beträgt p_{D} von 1,01 bar bis 22,5 bar, besonders bevorzugt 5,00 bar bis 17,5 bar. Das die Destillationskolonne (5000) verlassende Kopfprodukt (100) weist bevorzugt eine Temperatur T_{D} im Bereich von -50 °C bis 20 °C, bevorzugt von -30 °C bis -10 °C auf. Geeignete Destillationskolonnen (5000) sind dem Fachmann bekannt und werden beispielsweise in Dubbel, "Taschenbuch für den Maschinenbau", Springer, 2001, S. N 11 bis N 14, beschrieben. Es können auch mehrere Destillationskolonnen hintereinandergeschaltet werden. In diesem Fall bezeichnet p_{D} den Druck am Kopf der letzten Destillationskolonne.

Es ist im Rahmen der vorliegenden Erfindung bevorzugt, Schritt (iv) wie folgt durchzuführen:
Die Ströme (60) und (90) werden bevorzugt vereinigt, und der vereinigte Strom wird durch einen Wärmeaustauscher geführt, in dem ein flüssiger und ein gasförmiger Strom erhalten werden, welche beide in die als Destillationskolonne ausgestaltete Reinigungsapparatur (5000) geführt werden, und zwar bevorzugt der gasförmige Strom oberhalb des flüssigen. Bevorzugt erfolgt in diesem Wärmeaustauscher eine Abkühlung auf eine Temperatur im Bereich von 0 °C bis 50 °C, besonders bevorzugt im Bereich von 10 °C bis 30 °C.

Der in der Destillationskolonne (5000) am Kopf erhaltene gasförmige Strom wird dann durch einen anderen Wärmeaustauscher geführt, in welchem durch indirekte Abkühlung (mit einem von außen zugeführten Kühlmittel) auf eine Temperatur im Bereich von -50 °C bis 20 °C, besonders bevorzugt im Bereich von -30 °C bis -10 °C, eine partielle Verflüssigung dieses Stroms stattfindet. Die so erhaltene flüssige Phase wird wieder auf den Kopf der Destillationskolonne (5000) zurückgeführt. Die verbleibende gasförmige Phase (das gereinigte Chlorwasserstoffgas (100)) hat eine geringere Temperatur als die Ströme (60) und (90) und wird in den dem Verdichter (4000) nachgeschalteten Wärmeaustauscher geführt, wo sie der indirekten Kühlung der vereinigten Ströme (90) und (60) dient. Hierdurch wird der Gasstrom (100) erwärmt, jedoch nicht in seiner Zusammensetzung verändert.

Der nach dem erfindungsgemäßen Verfahren hergestellte Chlorwasserstoffstrom (100) zeichnet sich durch eine hohe Reinheit aus, sodass er auch für sensible Anwendungen wie den Einsatz in einem Deacon-Verfahren geeignet ist. Des Weiteren liegt der Chlorwasserstrom (100) bei einem Druck p_{D} vor, der höher ist als der für die Folgeanwendung angestrebte Druck p_{F}. Im Idealfall ist die Druckdifferenz nur so groß, dass beim Transport des Chlorwasserstoffs (100) zur Folgeanwendung der dort angestrebte Druck p_{F} infolge inhärenter Druckverluste ohne weitere Maßnahmen erreicht wird. Ist dies nicht der Fall, kann durch Einbau eines einfachen, dem Fachmann bekannten Druckminderers der gewünschte Druck p_{F} eingestellt werden.

Bevorzugte Folgeanwendungen des Chlorwasserstoffs (100) sind:
- Die katalytische Oxidation mit Sauerstoff zu Chlor ("Deacon-Prozess"), die bevorzugt bei einem Druck p_{F} von von 1,00 bar bis 25,0 bar, bevorzugt 1,50 bar bis 17,00 bar, besonders bevorzugt 1,5 bis 17 bar und insbesondere 2,0 bis 15 bar (absolut) durchgeführt wird. Der Deacon-Prozess ist dem Fachmann bekannt und bspw. beschrieben in Ullmann's Encyclopedia of Industrial Chemistry, 2012 Wiley-VCH Verlag GmbH & Co. KGaA, Weinheim, Kapitel 10.2.1 (S. 597 bis 599), sowie der dort zitierten Literatur. Dabei sind im Rahmen der vorliegenden Erfindung Katalysatoren auf Basis von Metalloxiden und/oder - chloriden (insbesondere der Metalle Ruthenium, Chrom, Zinn, Cer oder Mischungen von mindestens zwei dieser Metalle) gegenüber dem Einsatz anorganischer Säuren als Katalysatoren bevorzugt,
- Die Umsetzung zu Ethylendichlorid (EDC) durch Oxychlorierung von Ethan oder Ethylen, bevorzugt Ethylen, die bevorzugt bei einem Druck p_{F} von 1,00 bar bis 15,0 bar, bevorzugt von 4,00 bar bis 12,0 bar (absolut) durchgeführt wird. Der EDC-Prozess ist dem Fachmann bekannt und bspw. beschrieben in Ullmann's Encyclopedia of Industrial Chemistry, 2014 Wiley-VCH Verlag GmbH & Co. KGaA, Weinheim, Kapitel 1.3 (S. 12 bis 17), sowie der dort zitierten Literatur.

Das zuvor beschriebene Verfahren ist auch aus dem Grund von besonderer wirtschaftlicher Bedeutung, dass die immer größer werdenden Isocyanat-Produktionskapazitäten die Frage dringlicher machen, wie der zwangsläufig anfallende Chlorwasserstoff am Standort wirtschaftlich sinnvoll verwertet werden kann.

Durch die nachfolgenden Beispiele wird die Erfindung noch näher erläutert.

### Beispiele

Die im Folgenden dargestellten Beispiele basieren auf einer Prozesssimulation des jeweiligen stationären Betriebszustands, wie sie z. B. in U. Plöcker, R. Janowsky, H. Briesen, W. Marquardt, "Prozessanalyse und -synthese: Modellierung, Simulation und Optimierung", Kapitel 9 "Stationäre und dynamische Prozesssimulation" in "Chemische Technik", Winnacker, Küchler (Hrsg.), Band 2, 5. Aufl., Wiley-VCH-Verlag, Weinheim, 2004, S. 161ff. in ihren wesentlichen Zügen beschrieben wird. Zur numerischen Lösung des stationären Betriebszustands lagen dem Modell einerseits die Phasengleichgewichte der einzelnen Komponenten und zum anderen die Reaktionskinetik der Umsetzung von MDA mit Phosgen unter Einhaltung der Energie- und Massenbilanz zu Grunde.

72 t/h einer 55-massenprozentigen Lösung von Phosgen in Monochlorbenzol (MCB) und 95 t/h einer 42-massenprozentigen Lösung eines Gemisches von Di- und Polyaminen der Diphenylmethanreihe (MDA) in MCB werden zur Reaktion gebracht, wobei ein Rohproduktstrom 40 (p_{R}) erhalten wird. Je nach Reaktionsführung im Reaktor 1000 (vgl. FIG. 1) steht dieser Strom unter einem bestimmten Druck p_{R}. In jedem Fall werden nach Destillation und Verdichtung etwa 29,5 t/h eines HCl-Stroms 100 mit rund 1 ppm Phosgen erhalten, der den Anforderungen einer Folgeanwendung wie beispielsweise dem Deacon- oder EDC-Prozess genügt. Die Rückläufe/Sümpfe der einzelnen Verfahrensstufen werden dem Aufarbeitungs-Prozess des Isocyanates zurückgeführt.

### Beispiel 1 (erfindungsgemäß, p_{R} = 40 bar (absolut))

Nachfolgende Tabelle führt die Resultate auf. Stromangaben beziehen sich auf die Bezeichnungen aus FIG. 1.

| Strom | 50 | 60 | 70 | 80 | 90 | 100 | 110 |
|---|---|---|---|---|---|---|---|
| Index i | - | E | - | A | V | D | - |
| HCl / kg/h | 3465,6 | 18534,5 | - | 11754,3 | 11754,3 | 29423,0 | 865,8 |
| Phosgen / kg/h | 1222,0 | 776,9 | 0,1 | 1219,0 | 1219,0 | 0,03 | 1995,9 |
| MCB / kg/h | 81974,4 | 1647,8 | 77388,0 | 668,1 | 668,1 | - | 2315,9 |
| Roh-MDI¹⁾ | 58389,9 | - | 50446,5 | - | - | - | - |
| Gesamt / kg/h | 141586,3 | 20959,2 | 127834,6 | 13641,4 | 13641,4 | 29423,03 | 5177,6 |
| Tᵢ / °C | 114,6 | 130,6 | 189,7 | 50,0 | 131,9 | -22,6 | 9,8 |
| pᵢ / bar | 3,00 | 12,0 | 3,00 | 3,00 | 12,0 | 11,0 | 11,0 |

| | | | | | | | |
|---|---|---|---|---|---|---|---|
| 1) Roh-MDI bestehend aus MDI, Carbaminsäurechloriden und Carbodiimiden. | | | | | | | |

### Beispiel 2 (erfindungsgemäß, p_{R} = 20 bar (absolut))

Nachfolgende Tabelle führt die Resultate auf. Stromangaben beziehen sich auf die Bezeichnungen aus FIG. 1.

| Strom | 50 | 60 | 70 | 80 | 90 | 100 | 110 |
|---|---|---|---|---|---|---|---|
| Index i | - | E | - | A | V | D | - |
| HCl/kg/h | 3096,4 | 20992,7 | - | 9323,0 | 9323,0 | 29419,4 | 896,3 |
| Phosgen / kg/h | 987,5 | 963,0 | 0,1 | 1005,9 | 1005,9 | 0,03 | 1968,8 |
| MCB / kg/h | 81491,7 | 2164,2 | 75889,3 | 530,2 | 530,2 | - | 2694,4 |
| Roh-MDI¹⁾ | 56071,5 | 0,1 | 50418,4 | - | - | - | 0,1 |
| Gesamt / kg/h | 141647,1 | 24120,0 | 126307,8 | 10859,1 | 10859,1 | 29419,5 | 5559,6 |
| Tᵢ / °C | 125,4 | 123,0 | 189,8 | 50,0 | 131,8 | -22,6 | 10,9 |
| pᵢ / bar | 3,00 | 12,0 | 3,00 | 3,00 | 12,0 | 11,0 | 11,0 |

| | | | | | | | |
|---|---|---|---|---|---|---|---|
| 1) Roh-MDI bestehend aus MDI, Carbaminsäurechloriden und Carbodiimiden. | | | | | | | |

Die Beispiele zeigen, dass es gelingt, Chlorwasserstoff in der angestrebten Reinheit zu erhalten, ohne dass der gesamte im Isocyanatprozess gebildete Chlorwassserstoff (also die Summe aus Chlorwasserstoff in Strom 60 und Strom 80) verdichtet werden müsste. Dagegen muss im Verfahren gemäß EP 2 021 275 B1 der gesamte Chlorwasserstoffstrom verdichtet werden. Außerdem fallen Nebenkomponenten an, die entsorgt werden müssen.

## Patentansprüche

1. Verfahren zur Verfügbarmachung von bei der Herstellung eines Isocyanats durch Phosgenierung des korrespondierenden Amins (10), ggf. in Gegenwart eines Lösungsmittels (30), als Koppelprodukt anfallenden Chlorwasserstoffs für eine gewünschte Folgeanwendung mit einem angestrebten Eingangsdruck des dort einzusetzenden Chlorwasserstoffs (100) von p_{F}, umfassend die Schritte:
(i) ein- oder mehrstufige Entspannung des Rohprodukts der Phosgenierung (40), das unter dem Druck p_{R} steht, auf einen Druck p_{E} > p_{F}, wodurch ein gasförmiger, Chlorwasserstoff enthaltender Strom (60) und ein flüssiger, Isocyanat- und Phosgen sowie ggf. Lösungsmittel enthaltender Strom (50) gewonnen werden;
(ii) Abtrennung von Phosgen aus dem überwiegend Isocyanat- und Phosgen sowie ggf. Lösungsmittel enthaltenden Strom (50) bei einem Druck p_{A} < p_{F}, wobei ein gasförmiger, Phosgen- und Chlorwasserstoff enthaltender Strom (80) und ein Isocyanat sowie ggf. Lösungsmittel enthaltender Strom (70) erhalten werden;
(iii) Verdichtung des Phosgen- und Chlorwasserstoff enthaltenden Stroms (80) auf einen Druck p_{V} > p_{F}, wobei ein verdichteter Gasstrom (90) erhalten wird;
(iv) Reinigung des Chlorwasserstoff enthaltenden Stroms (60) und des verdichteten Phosgen- und Chlorwasserstoff enthaltenden Stroms (90), bei einem Druck p_{D} > p_{F}, wobei gereinigtes Chlorwasserstoffgas (100) und Phosgen-haltige Flüssigkeit (110) erhalten werden.

2. Verfahren nach Anspruch 1, bei dem Schritt (i) in einem Abscheider (2000) durchgeführt wird.

3. Verfahren nach Anspruch 2, bei dem der Abscheider (2000) als eine Kaskade mehrerer hintereinander geschalteter Abscheider (2010, 2020, 2030, ...) mit sukzessive sinkendem Druckniveau ausgestaltet ist, wobei jedem Abscheider eine gasförmige, Chlorwasserstoff enthaltende Phase (61, 62, 63, ...) entnommen wird, wobei ferner diese einzelnen gasförmigen, Chlorwasserstoff enthaltenden Phasen (61, 62, 63, ...) zum gasförmigen, Chlorwasserstoff enthaltenden Strom (60) vereinigt werden, dessen Druck dem Druck p_{E} entspricht.

4. Verfahren nach einem der vorstehenden Ansprüche, bei dem Schritt (ii) in einer Entphosgenierkolonne (3000) durchgeführt wird.

5. Verfahren nach einem der vorstehenden Ansprüche, bei dem Schritt (iv) in einer Destillationskolonne (5000) durchgeführt wird.

6. Verfahren nach einem der vorstehenden Ansprüche, bei dem die Ströme (60) und (90) vor Durchführung von Schritt (iv) vereinigt werden.

7. Verfahren nach den Ansprüchen 5 und 6, bei dem
(iv.1) der durch Vereinigung der Ströme (60) und (90) erhaltene Strom in einem Wärmeaustauscher durch indirekte Kühlung partiell kondensiert wird,
(iv.2) der so erhaltene flüssige Strom und der so erhaltene Gasstrom in die Destillationskolonne (5000) geleitetet werden,
(iv.3) der am Kopf der Destillationskolonne (5000) erhaltene gasförmige Strom in einem anderen Wärmeaustauscher durch indirekte Kühlung partiell kondensiert wird,
(iv.4) die in (iv.3) erhaltene Flüssigphase in den Kopf der Destillationskolonne (5000) zurückgeführt wird,
(iv.5) die in (iv.3) erhaltene Gasphase, das gereinigte Chlorwasserstoffgas (100), als Mittel zur indirekten Kühlung in Schritt (iv.1) eingesetzt wird.

8. Verfahren nach einem der vorstehenden Ansprüche, bei dem das Isocyanat ausgewählt ist aus der Gruppe bestehend aus Methylendiphenyldiisocyanat als reines Isomer oder als Isomerengemisch, Polymethylenpolyphenylpolyisocyanat, Gemische aus Methylendiphenyldiisocyanat und Polymethylenpolyphenylpolyisocyanat, Toluylendiisocyanat als reines Isomer oder Isomerengemisch, Isomere des Xylylendiisocyanats, Isomere des Diisocyanatobenzols, 2,6-Xylolisocyanat und 1,5-Naphthylendiisocyanat.

9. Verfahren nach einem der vorstehenden Ansprüche, bei dem
• p_{R} einen Wert im Bereich von 6,00 bar bis 60,0 bar,
• p_{E} einen Wert im Bereich von 5,00 bar bis 30,0 bar,
• p_{A} einen Wert im Bereich von 0,50 bar bis 5,00 bar,
• p_{V} einen Wert im Bereich von 5,00 bar bis 30,0 bar, und
• p_{D} einen Wert im Bereich von 1,01 bar bis 25,5 bar
aufweist.

10. Verfahren nach einem der Ansprüche 1 bis 8, bei dem
• p_{R} einen Wert im Bereich von 12,0 bar bis 45,0 bar,
• p_{E} einen Wert im Bereich von 9,00 bar bis 18,0 bar;
• p_{A} einen Wert im Bereich von 1,00 bar bis 3,00 bar;
• p_{V} einen Wert im Bereich von 9,00 bar bis 18,0 bar und
• p_{D} einen Wert im Bereich von 5,00 bar bis 17,5 bar
aufweist.

11. Verfahren nach Anspruch 9, bei dem
• das Rohprodukt der Phosgenierung (40) eine Temperatur T_{R} im Bereich von 80 °C bis 200 °C,
• der gasförmige, Chlorwasserstoff enthaltende Strom (60) eine Temperatur T_{E} im Bereich von 90 °C bis 170 °C,
• der gasförmiger, Phosgen- und Chlorwasserstoff enthaltende Strom (80) eine Temperatur T_{A} im Bereich von 10 °C bis 90 °C,
• der verdichteter Gasstrom (90) eine Temperatur Tv im Bereich von 90 °C bis 170 °C und
• das gereinigte Chlorwasserstoffgas (100) eine Temperatur T_{D} im Bereich von -50 °C bis 20 °C
aufweist.

12. Verfahren nach Anspruch 10, bei dem
• das Rohprodukt der Phosgenierung (40) eine Temperatur T_{R} im Bereich von 110 °C bis 170 °C,
• der gasförmige, Chlorwasserstoff enthaltende Strom (60) eine Temperatur T_{E} im Bereich von 110 °C bis 150 °C,
• der gasförmige, Phosgen- und Chlorwasserstoff enthaltende Strom (80) eine Temperatur T_{A} im Bereich von 30 °C bis 70 °C,
• der verdichteter Gasstrom (90) eine Temperatur T_{V} im Bereich von 110 °C bis 150 °C und
• das gereinigte Chlorwasserstoffgas (100) eine Temperatur T_{D} im Bereich von -30 °C bis - 10 °C
aufweist.

13. Verfahren nach einem der vorstehenden Ansprüche, bei dem die Folgeanwendung ausgewählt ist aus der Herstellung von Chlor durch katalytische Oxidation von Chlorwasserstoff mit Sauerstoff und der Umsetzung zu Ethylendichlorid durch Oxychlorierung von Ethan oder Ethylen.

14. Verfahren nach einem Ansprüche 9 oder 11, bei dem die Folgeanwendung ausgewählt ist aus
der Herstellung von Chlor durch katalytische Oxidation von Chlorwasserstoff mit Sauerstoff mit einem Wert für p_{F} im Bereich von 1,00 bar bis 25,0 bar, und
der Umsetzung zu Ethylendichlorid durch Oxychlorierung von Ethan oder Ethylen mit einem Wert für p_{F} im Bereich von 1,00 bar bis 15,0 bar.

15. Verfahren nach einem Ansprüche 10 oder 12, bei dem die Folgeanwendung ausgewählt ist aus
der Herstellung von Chlor durch katalytische Oxidation von Chlorwasserstoff mit Sauerstoff mit einem Wert für p_{F} im Bereich von 1,50 bar bis 17,0 bar,
der Umsetzung zu Ethylendichlorid durch Oxychlorierung von Ethan oder Ethylen mit einem Wert für p_{F} im Bereich von 4,00 bar bis 12,0 bar.

## Claims

1. Process for making hydrogen chloride obtained as coproduct in the preparation of an isocyanate by phosgenation of the corresponding amine (10), optionally in the presence of a solvent (30), available for a desired subsequent use at a desired admission pressure of the hydrogen chloride (100) to be used there of p_{F}, which comprises the steps:
(i) single-stage or multistage depressurization of the crude product from the phosgenation (40), which is under the pressure p_{R}, to a pressure p_{E} > p_{F}, giving a gaseous stream (60) containing hydrogen chloride and a liquid stream (50) containing isocyanate and phosgene and also optionally solvent;
(ii) separation of phosgene from the stream (50) containing predominantly isocyanate and phosgene and also optionally solvent at a pressure p_{A} < p_{F}, giving a gaseous stream (80) comprising phosgene and hydrogen chloride and a stream (70) containing isocyanate and optionally solvent;
(iii) compression of the stream (80) containing phosgene and hydrogen chloride to a pressure p_{V} > p_{F}, giving a compressed gas stream (90);
(iv) purification of the stream (60) containing hydrogen chloride and of the compressed stream (90) containing phosgene and hydrogen chloride at a pressure p_{D} > p_{F}, giving purified hydrogen chloride gas (100) and phosgene-containing liquid (110) .

2. Process according to Claim 1, wherein step (i) is carried out in a separator (2000).

3. Process according to Claim 2, wherein the separator (2000) is configured as a cascade of a plurality of separators (2010, 2020, 2030, ...) connected in series with a successively decreasing pressure level, where a gaseous, hydrogen chloride-containing phase (61, 62, 63...) is taken off from each separator and these individual gaseous phases (61, 62, 63, ...) containing hydrogen chloride are further combined to form the gaseous stream (60) containing hydrogen chloride, the pressure of which corresponds to the pressure p_{E}.

4. Process according to any of the preceding claims, wherein step (ii) is carried out in a dephosgenation column (3000).

5. Process according to any of the preceding claims, wherein step (iv) is carried out in a distillation column (5000).

6. Process according to any of the preceding claims, wherein the streams (60) and (90) are combined before step (iv) is carried out.

7. Process according to Claims 5 and 6, wherein
(iv.1) the stream obtained by combining the streams (60) and (90) is partially condensed by indirect cooling in a heat exchanger,
(iv.2) the resulting liquid stream and the resulting gas stream are fed into the distillation column (5000),
(iv.3) the gaseous stream obtained at the top of the distillation column (5000) is partially condensed by indirect cooling in another heat exchanger,
(iv.4) the liquid phase obtained in (iv.3) is recirculated to the top of the distillation column (5000),
(iv.5) the gas phase obtained in (iv.3), viz. the purified hydrogen chloride gas (100), is used as medium for indirect cooling in step (iv.1).

8. Process according to any of the preceding claims, wherein the isocyanate is selected from the group consisting of methylenedi(phenyl isocyanate) as pure isomer or as isomer mixture, polymethylenepolyphenyl polyisocyanate, mixtures of methylenedi(phenyl isocyanate) and polymethylenepolyphenyl polyisocyanate, tolylene diisocyanate as pure isomer or isomer mixture, isomers of xylylene diisocyanate, isomers of diisocyanatobenzene, 2,6-xylene isocyanate and naphthylene 1,5-diisocyanate.

9. Process according to any of the preceding claims, wherein
• p_{R} has a value in the range from 6.00 bar to 60.0 bar,
• p_{E} has a value in the range from 5.00 bar to 30.0 bar,
• p_{A} has a value in the range from 0.50 bar to 5.00 bar,
• p_{V} has a value in the range from 5.00 bar to 30.0 bar and
• p_{D} has a value in the range from 1.01 bar to 25.5 bar.

10. Process according to any of Claims 1 to 8, wherein
• p_{R} has a value in the range from 12.0 bar to 45.0 bar,
• p_{E} has a value in the range from 9.00 bar to 18.0 bar;
• p_{A} has a value in the range from 1.00 bar to 3.00 bar;
• p_{V} has a value in the range from 9.00 bar to 18.0 bar and
• p_{D} has a value in the range from 5.00 bar to 17.5 bar.

11. Process according to Claim 9, wherein
• the crude product from the phosgenation (40) has a temperature T_{R} in the range from 80°C to 200°C,
• the gaseous stream (60) containing hydrogen chloride has a temperature T_{E} in the range from 90°C to 170°C,
• the gaseous stream (80) containing phosgene and hydrogen chloride has a temperature T_{A} in the range from 10°C to 90°C,
• the compressed gas stream (90) has a temperature T_{V} in the range from 90°C to 170°C and
• the purified hydrogen chloride gas (100) has a temperature T_{D} in the range from -50°C to 20°C.

12. Process according to Claim 10, wherein
• the crude product form the phosgenation (40) has a temperature T_{R} in the range from 110°C to 170°C,
• the gaseous stream (60) containing hydrogen chloride has a temperature T_{E} in the range from 110°C to 150°C,
• the gaseous stream (80) containing phosgene and hydrogen chloride has a temperature T_{A} in the range from 30°C to 70°C,
• the compressed gas stream (90) has a temperature T_{V} in the range from 110°C to 150°C and
• the purified hydrogen chloride gas (100) has a temperature T_{D} in the range from -30°C to -10°C.

13. Process according to any of the preceding claims, wherein the subsequent use is selected from among the preparation of chlorine by catalytic oxidation of hydrogen chloride by means of oxygen and the reaction to form ethylene dichloride by oxychlorination of ethane or ethylene.

14. Process according to either Claim 9 or 11, wherein the subsequent use is selected from among the preparation of chlorine by catalytic oxidation of hydrogen chloride by means of oxygen at a value of p_{F} in the range from 1.00 bar to 25.0 bar and the reaction to form ethylene dichloride by oxychlorination of ethane or ethylene at a value of p_{F} in the range from 1.00 bar to 15.0 bar.

15. Process according to either Claim 10 or 12, wherein the subsequent use is selected from among the preparation of chlorine by catalytic oxidation of hydrogen chloride by means of oxygen at a value of p_{F} in the range from 1.50 bar to 17.0 bar,
the reaction to form ethylene dichloride by oxychlorination of ethane or ethylene at a value of p_{F} in the range from 4.00 bar to 12.0 bar.

## Revendications

1. Procédé pour la mise à disposition de chlorure d'hydrogène généré en tant que coproduit dans la préparation d'un isocyanate par la phosgénation de l'amine correspondante (10), éventuellement en présence d'un solvant (30), pour une application subséquente souhaitée à une pression d'entrée recherchée p_{F} du chlorure d'hydrogène qui y sera appliquée (100), comprenant les étapes de :
(i) détente en une ou plusieurs étape (s) du produit brut de la phosgénation (40), qui se trouve sous la pression p_{R}, à une pression p_{E} > p_{F}, ce qui permet d'obtenir un flux (60) gazeux contenant du chlorure d'hydrogène et un flux (50) liquide contenant de l'isocyanate et du phosgène ainsi qu'éventuellement un solvant ;
(ii) séparation du phosgène du flux (50) contenant majoritairement de l'isocyanate et du phosgène ainsi qu'éventuellement un solvant à une pression p_{A} < p_{F}, un flux (80) gazeux contenant du phosgène et du chlorure d'hydrogène et un flux (70) contenant de l'isocyanate ainsi qu'éventuellement un solvant étant obtenus ;
(iii) compression du flux (80) contenant du phosgène et du chlorure d'hydrogène à une pression p_{V} > p_{F}, un flux de gaz comprimé (90) étant obtenu ;
(iv) purification du flux (60) contenant du chlorure d'hydrogène et du flux (90) comprimé contenant du phosgène et du chlorure d'hydrogène, à une pression P_{D} > p_{F}, du chlorure d'hydrogène purifié (100) et un liquide (110) contenant du phosgène étant obtenus.

2. Procédé selon la revendication 1, l'étape (i) étant mise en oeuvre dans un séparateur (2000).

3. Procédé selon la revendication 2, dans lequel le séparateur (2000) est conçu en tant que cascade de plusieurs séparateurs (2010, 2020, 2030...) disposés en série présentant des niveaux de pression successifs décroissants, une phase gazeuse (61, 62, 63...) contenant du chlorure d'hydrogène étant prélevée de chaque séparateur, ces phases (61, 62, 63...) gazeuses individuelles contenant du chlorure d'hydrogène étant en outre rassemblées pour former le flux (60) gazeux contenant du chlorure d'hydrogène, dont la pression correspond à la pression p_{E}.

4. Procédé selon l'une quelconque des revendications précédentes, dans lequel l'étape (ii) est mise en oeuvre dans une colonne de déphosgénation (3000).

5. Procédé selon l'une quelconque des revendications précédentes, dans lequel l'étape (iv) est mise en oeuvre dans une colonne de distillation (5000).

6. Procédé selon l'une quelconque des revendications précédentes, dans lequel les flux (60) et (90) sont réunis avant la mise en oeuvre de l'étape (iv) .

7. Procédé selon les revendications 5 et 6, dans lequel
(iv.1) le flux obtenu par le rassemblement des flux (60) et (90) est partiellement condensé dans un échangeur thermique par refroidissement indirect,
(iv.2) le flux liquide ainsi obtenu et le flux gazeux ainsi obtenu sont acheminés dans la colonne de distillation (5000),
(iv.3) le flux gazeux obtenu à la tête de la colonne de distillation (5000) est partiellement condensé dans un autre échangeur thermique par refroidissement indirect,
(iv.4) la phase liquide obtenue en (iv.3) est ramenée dans la tête de la colonne de distillation (5000),
(iv.5) la phase gazeuse obtenue en (iv.3), le chlorure d'hydrogène gazeux purifié (100), est utilisée comme agent pour le refroidissement indirect dans l'étape (iv.1).

8. Procédé selon l'une quelconque des revendications précédentes, l'isocyanate étant choisi dans le groupe constitué par le diisocyanate de diphénylméthylène, en tant qu'isomère pur ou en tant que mélange d'isomères, le polyisocyanate de polyphényle polyméthylène, les mélanges de diisocyanate de diphénylméthylène et de polyisocyanate de polyphényle polyméthylène, le diisocyanate de toluylène en tant qu'isomère pur ou en tant que mélange d'isomères, les isomères du diisocyanate de xylylène, les isomères du diisocyanatobenzène, l'isocyanato-2,6-xylène et le 1,5-diisocyanate de naphtalène.

9. Procédé selon l'une quelconque des revendications précédentes, dans lequel
• p_{R} présente une valeur dans la plage de 6,00 bars jusqu'à 60,0 bars,
• p_{E} présente une valeur dans la plage de 5,00 bars jusqu'à 30,0 bars,
• p_{A} présente une valeur dans la plage de 0,50 bar jusqu'à 5,00 bars,
• p_{V} présente une valeur dans la plage de 5,00 bars jusqu'à 30,0 bars et
• p_{D} présente une valeur dans la plage de 1,01 bar jusqu'à 25,5 bars.

10. Procédé selon l'une quelconque des revendications 1 à 8, dans lequel
• p_{R} présente une valeur dans la plage de 12,0 bars jusqu'à 45,0 bars,
• p_{E} présente une valeur dans la plage de 9,00 bars jusqu'à 18,0 bars,
• p_{A} présente une valeur dans la plage de 1,00 bar jusqu'à 3,00 bars,
• p_{V} présente une valeur dans la plage de 9,00 bars jusqu'à 18,0 bars et
• p_{D} présente une valeur dans la plage de 5,00 bars jusqu'à 17,5 bars.

11. Procédé selon la revendication 9, dans lequel
• le produit brut de la phosgénation (40) présente une température T_{R} dans la plage de 80°C jusqu'à 200°C,
• le flux gazeux contenant du chlorure d'hydrogène (60) présente une température T_{E} dans la plage de 90°C jusqu'à 170°C,
• le flux gazeux contenant du phosgène et du chlorure d'hydrogène (80) présente une température T_{A} dans la plage de 10°C jusqu'à 90°C,
• le flux de gaz comprimé (90) présente une température T_{V} dans la plage de 90°C jusqu'à 170°C et
• le chlorure d'hydrogène gazeux purifié (100) présente une température T_{D} dans la plage de -50°C jusqu'à 20°C.

12. Procédé selon la revendication 10, dans lequel
• le produit brut de la phosgénation (40) présente une température T_{R} dans la plage de 110°C jusqu'à 170°C,
• le flux gazeux contenant du chlorure d'hydrogène (60) présente une température T_{E} dans la plage de 110°C jusqu'à 150°C,
• le flux gazeux contenant du phosgène et du chlorure d'hydrogène (80) présente une température T_{A} dans la plage de 30°C jusqu'à 70°C,
• le flux de gaz comprimé (90) présente une température T_{V} dans la plage de 110°C jusqu'à 150°C et
• le chlorure d'hydrogène gazeux purifié (100) présente une température T_{D} dans la plage de -30°C jusqu'à -10°C.

13. Procédé selon l'une quelconque des revendications précédentes, dans lequel l'application subséquente est choisie parmi la préparation de chlore par oxydation catalytique de chlorure d'hydrogène avec de l'oxygène et la transformation d'éthane ou d'éthylène en dichlorure d'éthylène par oxychloration.

14. Procédé selon l'une quelconque des revendications 9 ou 11, dans lequel l'application subséquente est choisie parmi la préparation de chlore par oxydation catalytique de chlorure d'hydrogène avec de l'oxygène, présentant une valeur de p_{F} se situant dans la plage de 1,00 bar jusqu'à 25,0 bars et
la transformation d'éthane ou d'éthylène en dichlorure d'éthylène par oxychloration, présentant une valeur de p_{F} se situant dans la plage de 1,00 bar jusqu'à 15,0 bars.

15. Procédé selon l'une quelconque des revendications 10 ou 12, dans lequel l'application subséquente est choisie parmi la préparation de chlore par oxydation catalytique de chlorure d'hydrogène avec de l'oxygène, présentant une valeur de p_{F} se situant dans la plage de 1,50 bar jusqu'à 17,0 bars et
la transformation d'éthane ou d'éthylène en dichlorure d'éthylène par oxychloration, présentant une valeur de p_{F} se situant dans la plage de 4,00 bars jusqu'à 12,0 bars.
